# EUROPEAN PATENT SPECIFICATION

(11) **EP 2 916 817 B1**
(45) Date of publication and mention of the grant of the patent: **12.09.2018**
(21) Application number: 13812219.7
(22) Date of filing: 12.11.2013
(51) Int. Cl.: A61K 9/08, A61K 31/045, A61P 23/02

(54) **AQUEOUS BASED CAPSAICINOID FORMULATIONS AND METHODS OF MANUFACTURE AND USE**
WASSERBASIERTE CAPSAICINOIDFORMULIERUNGEN UND VERFAHREN ZUR HERSTELLUNG UND VERWENDUNG
FORMULATIONS DE CAPSAÏCINOÏDE À BASE AQUEUSE ET PROCÉDÉS DE FABRICATION ET D'UTILISATION

(30) Priority: 12.11.2012 US 201261725161 P
(43) Date of publication of application: 16.09.2015
(60) Divisional application: 18182795.7
(73) Proprietor: Vizuri Health Sciences LLC, Fairfax, VA 22033 (US)
(72) Inventor: BIRBARA, Charles, A., Worcester, MA 01609 (US); BIRBARA, Philip, J., deceased (US); BUCKS, Daniel, Millbrae, CA 94030 (US); COUGHLIN, Mary, Worcester, MA 01606 (US)
(74) Representative: Stafford, Jonathan Alan Lewis
(86) International application number: PCT/US2013/069711
(87) International publication number: WO 2014/075084

(56) References cited:
- US-A- 4 599 342
- US-A- 4 702 916
- US-A1- 2005 019 436
- US-A1- 2008 153 780
- US-B1- 6 573 302

## Description

### Field of the Invention

The present invention is directed to compositions for the administration of a capsaicinoid into localized areas. The compositions are useful for the treatment of multiple disorders involving pain (acute and chronic, moderate to severe), and they provide extended pain relief. The invention also relates to methods including pretreatments which limit burning and stinging pain associated with capsaicinoid injection.

### Background of the Invention

Capsaicin, a pungent substance in the fruit of capsicum plants, works to relieve pain by causing a localized degradation of the C neuron endings; capsaicinoids being the only analgesics known to relieve pain by this mechanism. The activity of capsaicin results from its binding to, and activating, an ion channel called vanilloid receptor 1 (VR1). Under normal circumstances, when the VR1 ion channel is activated it opens for a short time, causing the C neurons to transmit a pain signal toward the brain. When capsaicin binds to, and activates VR1, it causes a series of events within the cell that degrade the C neural endings, or terminals of the C neuron, thereby preventing the neuron from transmitting pain signals.

Although capsaicin's analgesic effect was thought to be due to depletion of the substance P, recent evidence suggests a process of "defunctionalization" of nociceptor fibers as being responsible for the analgesic effect of capsaicin. **(**Anand P, BleyK. Topical capsaicin for pain management: therapeutic potential and mechanisms of action of the new high-concentration capsaicin 8% patch. Br J Anaesth. 2011;107(4):490-502***.)***

Capsaicin topical ointments and creams relieve minor aches and pains of muscles and joints. Capsaicin is currently marketed as over-the-counter topically applied, non-sterile creams and patches containing capsaicin at low doses. Concentrations of capsaicin are typically between 0.025 wt.% and 0.075 wt.%. These over-the-counter topical preparations are used topically by consumers to relieve pain with variable and often inadequate results when used to treat conditions such as osteoarthritis, shingles (herpes zoster), psoriasis and diabetic neuropathy. Capsaicin is also available in large adhesive bandages that can be applied to the back.

Topical preparations of capsaicin are used in a variety of skin disorders that involve pain and itching, such as postherpetic neuralgia, diabetic neuropathy, pruritus, psoriasis, cluster headache, postmastectomy pain syndrome, rhinopathy, oral mucositis, cutaneous allergy, detrusor hyperreflexia, loin pain/hematuria syndrome, neck pain, amputation stump pain, reflex sympathetic dystrophy, skin tumor, arthritis including rheumatoid arthritis and osteoarthritis, post-surgical pain, oral pain, and pain caused by injury, amongst others. *(*Martin Hautkappe et al., Review of the Effectiveness of Capsaicin for Painful Cutaneous Disorders and Neural Dysfunction, Clin. J. Pain, 14:97-106, 1998*).*

Anti-inflammatories decrease the inflammation and swelling at the tissue site. They do this by blocking certain physiologically active substances. For example, these substances, prostaglandin, substance P and histamine, can cause small arteries to dilate with subsequent edema or fluid formation. Soft tissue, muscle, and nerve cells become irritable inflamed and hyper excitable. Anti-inflammatory medications can inhibit this inflammatory process. The treatment of chronic pain with anti-inflammatories however, is limited.

The mainstays for pain relief after total hip arthroplasty and total knee arthroplasty have been the opioids. Although these medications are excellent analgesics, opioids have problems that limit their effectiveness. Alternative analgesics have been considered too mild for the pain caused by these procedures.

US Patent 4,599,342 discloses an analgesic composition combining capsaicin or a capsaicin analogue with an analgesic selected from the class of opioids.

US Patent 4,702,916 discloses an analgesic stick composition comprising a delivery system comprising alcohol, an alkali metal salt of a saturated fatty acid, water and an analgesic compound selected from the group consisting of oleoresin capsicum, capsicum, capsaicin, camphor, allyl isothiocyanate, methyl nicotinate, menthol and mixtures thereof.

US Patent 5962532 discloses methods and compositions for treating pain at a specific site with an effective concentration of capsaicin or analogues thereof. The methods involve providing anesthesia to the site where the capsaicin or analogues thereof is to be administered, and then administering an effective concentration of capsaicin to the joint.

US Patent 6,573,302 discloses a cream comprising a topical carrier selected from preservatives, an encapsulation agent, esters of amino acids, a light scattering element and a histidine.

US Patent 8,158,682 relates to methods for treating or attenuating pain in a patient. Specifically, the invention provides a method for attenuating pain in proximity to the site of an open wound or surgical incision comprising instilling a pharmaceutical composition comprising a capsaicinoid into the wound or incision, allowing the pharmaceutical composition to dwell for a predetermined period of time, and aspirating the wound or incision to remove the pharmaceutical composition. The invention also provides a method for attenuating pain in proximity to a joint comprising intra-articularly injecting a pharmaceutical composition comprising a capsaicinoid into the joint, allowing the pharmaceutical composition to dwell for predetermined period of time, and aspirating the joint to remove the pharmaceutical composition. In certain embodiments of the invention, the capsaicinoid is capsaicin.

US Patent 8,420,600 discloses compositions and methods for relieving pain at a site in a human or animal by administering at a discrete site in a human or animal a dose of capsaicin in an amount effective to denervate the discrete site without eliciting an effect outside the discrete location.

US 2004/0161481 discloses a method for relieving pain at a site in a human or animal in need thereof, comprising administering by injection or infiltration, a dose of a capsaicinoid and coadministering an effective amount of a NSAID to decrease an undesired effect of the capsaicinoid.

US 2004/0156931discloses a method for relieving pain at a site in a human or animal in need thereof, comprising administering by injection or infiltration, a dose of a capsaicinoid and coadministering a vasodilator.

US 2004/0186182 discloses a method for relieving pain at a site in a human or animal in need thereof, comprising administering by injection or infiltration, a dose of a capsaicinoid and coadministering a non-anesthetic sodium channel blocker.

US 2005/0019436 discloses compositions and methods for relieving pain at a site in a human or animal in need thereof by administering at a discrete site in a human or animal in need thereof a dose of capsaicin in an amount effective to denervate a discrete site without eliciting an effect outside the discrete location, the dose of capsaicin ranging from 1 µg to 3000 µg.

US 2005/0020690 discloses compositions and methods for attenuating or relieving pain at a site in a human or animal in need thereof by infiltrating at a surgical site or open wound in a human or animal a dose of capsaicinoid in an amount effective to denervate the surgical site or open wound substantially without eliciting an effect outside the surgical site or open wound.

US 2005/0058734 discloses a method for relieving pain at a site in a human or animal in need thereof, comprising administering by injection or infiltration, a dose of a capsaicinoid and coadministering a vasoconstrictor.

US 2006/0269628 discloses compositions and methods for attenuating or relieving pain at a site in a human or animal in need thereof by infiltrating at a surgical site or open wound in a human or animal a dose of capsaicinoid in an amount effective to denervate the surgical site or open wound substantially without eliciting an effect outside the surgical site or open wound.

U.S. Patent Application 2006/0100272 discloses compositions and methods for the treatment of pain, and neuropathic pain in particular.

US 2006/0148903 provides capsaicinoid gel formulations and methods for relieving pre- and post-surgical pain at a site in a human or animal by administering at a surgical site in a human or animal in need thereof a dose of capsaicinoid gel in an amount effective to attenuate post-surgical pain at the surgical site, the dose of capsaicin ranging from 100 µg to 10,000 µg.

US 2007/0293703 provides methods for synthesizing the trans isomer of capsaicin and/or capsaicin-like compounds by utilizing a process wherein the trans geometry is set from the beginning of the synthesis reaction and carried through the entire synthesis process.

US 2008/0153780 relates to the use of a vanilloid receptor agonist together with a glycosaminoglycan for producing an agent for treating pain.

US 2007/0036876 provides compositions and methods for relieving pain at a site in a human or animal in need thereof by administering at a discrete site in a human or animal in need thereof a dose of capsaicin in an amount effective to denervate a discrete site without eliciting an effect outside the discrete location, the dose of capsaicin ranging from 1 µg to 3000 µg.

US 2008/0260791 provides compositions and methods for relieving pain at a site in a human or animal in need thereof by administering at a discrete site in a human or animal in need thereof a dose of capsaicin in an amount effective to denervate a discrete site without eliciting an effect outside the discrete location, the dose of capsaicin ranging from 1 µg to 5000 µg.

US 2008/0262091 A1 provides compositions and methods for attenuating or relieving pain at a site in a human or animal in need thereof by infiltrating at a surgical site or open wound in a human or animal a dose of capsaicinoid in an amount effective to denervate the surgical site or open wound substantially without eliciting an effect outside the surgical site or open wound.

US 2009/0062359 relates to a method for relieving pain at a site in a human or animal in need thereof, comprising administering by injection or infiltration, a dose of a capsaicinoid and coadministering a non-anesthetic sodium channel blocker.

US 2009/0054527 discloses a method for relieving pain at a site in a human or animal in need thereof, comprising administering by injection or infiltration, a dose of a capsaicinoid and coadministering a vasodilator.

US 2009/0111792 discloses a method for relieving pain at a site in a human or animal in need thereof, comprising administering by injection or infiltration, a dose of a capsaicinoid and coadministering a tricyclic antidepressant.

US 2009/0117167 A1 discloses a method for relieving pain at a site in a human or animal in need thereof, comprising administering by injection or infiltration, a dose of a capsaicinoid and coadministering a vasoconstrictor.

US 2009/0118242 discloses a method for relieving pain at a site in a human or animal in need thereof, comprising administering by injection or infiltration, a dose of a capsaicinoid and coadministering an effective amount of a NSAID to decrease an undesired effect of the capsaicinoid.

US 2011/0311592 teaches methods of increasing solubility of poorly soluble compounds and methods of making and using formulations of such compounds.

ALGRX-4975 was in clinical developed to treat the pain associated with osteoarthritis, tendonitis and postsurgical conditions, as well as for neuropathic pain occurring secondary to nerve injury and other chronic pain conditions. By targeting only the C neuron pain fibers, ALGRX-4975 was able to produce significant long-term analgesia. In clinical studies, ALGRX-4975 has provided long-term relief of pain from a single treatment as summarized in TABLE I.

**TABLE I - A SUMMARY OF ALGRX-4975 CLINICAL TRAILS**

| **ALGRX-4975** | | **COMMENTS** |
|---|---|---|
| **Volume (ml)** | **Concentration (mg/ml)** | |
| 4 | 0.25 | ⁽¹⁾Significant pain reduction of mean Visual Analogue Scale (VAS) at 8 hr and 24 hr post unilateral bunionectomy after a single intra-operative instillation. |
| 0.5 | 0.2 | ⁽²⁾Significant reduction in pain of intermetatarsal neuroma at week 1 and 4 compared to placebo |
| 0.5 | 0.2 | ⁽²⁾Lowered pain scores in patients with end-stage OA of the knee waiting for knee replacement |
| 15 | 0.067 | ⁽³⁾During inguinal hernia repair improved analgesia relative to placebo following the first 3-4 days post surgery |

| | | |
|---|---|---|
| ⁽¹⁾Cantilon, M. et al, Preliminary safety, tolerability and efficacy of ALGRX 4975 in Osteoarthritis (OA) of the knee, The Journal of Pain, Vol. 6, March, 2005. ⁽²⁾Diamond, E. et al, ALGRX 4975 reduces pain of intermetatarsal neuroma: preliminary results from a randomized, double-blind, placebo-controlled, phase II multicenter clinical trial, The Journal of Pain, Vol. 7, April, 2006. ⁽³⁾Aasvang, E. et al, The effect of wound instillation of a novel purified capsaicin formulation on postherniotomy pain: a double-blind, randomized, placebo-controlled study, Anesthesia and Analgesia, 2008, 107(1), p.282-291 | | |

For several clinical trials, a high purity trans-capsaicin was supplied in vials containing 5 mL of purified capsaicin at concentrations of 0.5 mg/ml dissolved in PEG-300. The capsaicin /PEG-300 concentrate was stored at a temperature between 15 °C and 25 °C. Within four hours prior to injection, the capsaicin concentrate was diluted to make a formulation comprising about 20% PEG-300, about 1.5 mg/ml histidine and about 5% sucrose. As noted in Table II, the capsaicin concentrations of the 3 listed examples in US Patent 8,420,600 are 0.002 mg/ml, 0.02 mg/ml and 0.06 mg/ml.

**TABLE II - Injectable Capsaicin Dose Levels**

| **Capsaicin Dose Level (mg)** | **Capsaicin Dose Concentration (mg/ml)** | **Total Volume of Injectable Dose (ml)** |
|---|---|---|
| **0.01** | **0.002** | **5** |
| **0.1** | **0.02** | **5** |
| **0.3** | **0.06** | **5** |

Upon capsaicin injection in clinical trials, spontaneous burning pain and hyperalgesia was experienced immediately and persisted for up to 60 minutes. These undesirable side effects were attributed to intense activation and temporary sensitization of the peripheral nociceptors at the site of capsaicin application. This activation and sensitization occur prior to the desensitization phase. In an attempt to control burning and stinging upon capsaicin injection, local anesthetics were injected and then withdrawn, prior to capsaicin injection.

A present limitation on the use of injectable capsaicin formulations is the likelihood of intense burning and stinging (B&S) pain for up to 1 hour and mild pain for another 1 -2 hours. The expected B&S of the capsaicin dose are believed to be from the intense nociceptor discharge occurring during the excitatory phase before nociceptor desensitization.

Work in many models has shown that capsaicin can activate sensory nerves to induce release of neuropeptides, in particular calcitonin gene-related peptide (CGRP) and the inflammatory substance P (SP)Lundberg et al, Co-existence of substance P and calcitonin gene-related peptide-like immunoreactivities in sensory nerves in relation to cardiovascular and bronchoconstrictor effects of capsaicin. Eur. J. Pharmacol., 108, 315-319, (1985**).** Martling et al, Calcitonin gene-related peptide and the lung: neuronal coexistence with substance P, release by capsaicin and vasodilatory effect Regul. Pept., 20, 125-139, (1988**).**

It has been demonstrated that capsaicin induced edema is insensitive to treatment with the anti-inflammatory steroid, dexamethasone, which is in contrast to other forms of inflammatory edema formation. Newbold et al, An Investigation into the mechanism of capsaicin-induced edema in rabbit skin, Brit. J. of Pharma., 114, 570-577, (1995).

There is an unmet need for injectable capsaicin formulations and pain treatment procedures that minimize the acute burning sensation produced following capsaicin injection. Clinical studies conducted with capsaicinoid injections have shown the ability of capsaicin to reduce pre and postsurgical pain, pain caused by osteoarthritis, tendonitis and other surgical procedures. Many current pain therapies have a slow onset of action. Pain ailments are most often managed with opioids and NSAIDs and chronic use is often limited by side effects. There is an unmet need for safe and effective therapies to treat chronic pain.

### Objects of the Invention

It is an object of the invention to devise formulations and methods for providing pain relief in mammals via injectable capsaicinoids that overcome the intense burning pain experienced with the administration of a capsaicinoid.

A further object of the subject invention is to provide formulations and methods of use to ameliorate or prevent the burning or stinging associated with capsaicinoid injection administration.

It is another objective of the invention to provide an optically clear solution containing aqueous and lipophilic ingredients which are totally miscible.

It is another objective of the present invention to provide formulation and methods for extended pain relief without sedation or anesthesia such as nerve, spinal or epidural blocks, for a range of therapeutic applications.

### Summary of the Invention

The subject invention includes aqueous pharmaceutical compositions comprising:
i) 0.0002% - 0.1% by weight of a capsaicinoid,
ii) 0.01% - 1% by weight of menthol and/or eugenol, and
iii) an aqueous vehicle comprising an amount of polyethylene glycol and/or ethyl alcohol to solubilize the capsaicinoid and analgesic agent in water,
   said composition further comprising 0.1% to 1.5% by weight of hyaluronic acid.

The aqueous vehicle may comprise:
i) 15 - 50 % by weight polyethylene glycol, and/or
ii) 0 - 40% ethyl alcohol. Optionally, the composition includes 0.1% - 1.25% by weight of hyaluronic acid. Advantageous amounts of the components are:
   0.005 - 0.03 % by weight capsaicin,
   0.01 - 0.1% by weight phenol,
   0.01 - 0.1% by weight menthol,
   20 - 35% by weight polyethylene glycol,
   5 - 15% by weight ethyl alcohol, and
   0.2 - 1% by weight hyaluronic acid.

Also disclosed is an aqueous pharmaceutical composition comprising:
i) 0.0002% - 0.05% by weight of a capsaicinoid,
ii) 0.001%-2.5% by weight of a nonionic surfactant,
iii) 0.01% - 0.5% by weight of an analgesic agent, and
iv) an aqueous vehicle comprising an amount ethyl alcohol to solubilize the capsaicinoid and analgesic agent in water.

Advantageously, the nonionic surfactant may be polysorbate 80, and the weight ratio of capsaicin to polysorbate 80 may be 1 to 5. Advantageously, the capsaicinoid may be solubilized by the formation of a concentrate with the nonionic surfactant. The analgesic agent is menthol and/or eugenol. In one embodiment, the composition further comprises an anesthetic agent. Advantageously, the aqueous vehicle comprises an amount of polyethylene glycol and ethyl alcohol to solubilize the capsaicinoid and analgesic agent in water. Optionally, the composition further comprises 0.1% - 1.25% by weight of hyaluronic acid. In a composition of the present disclosure, advantageous amounts of the components may be:
0.005 - 0.03% by weight capsaicin,
0.025 - 0.15% by weight polysorbate 80,
0.01 - 0.1% by weight phenol,
0.01 - 0.1% by weight menthol,
5 - 15 % by weight polyethylene glycol 300 or 400,
5 - 15% by weight ethyl alcohol, and
0.2 - 1% by weight hyaluronic acid.

Also disclosed is an aqueous pharmaceutical composition comprising:
i) 0.0002% - 0.1% by weight of a capsaicinoid,
ii) 0.001%-1.0% by weight of a nonionic surfactant, and
iii) 0.01% - 1.0% by weight of an analgesic agent.

Advantageously, the nonionic surfactant may be is Cremophor® RH 40 (Polyoxyl 40 Hydrogenated Castor Oil; alternatively, Cremophor® ELP, or Solute® HS 15), and the weight ratio of capsaicin to Cremophor® RH 40 can vary from 1/1 to 1/6. Advantageously, the capsaicinoid may be solubilized by the formation of an anhydrous concentrate with the nonionic surfactant. The analgesic agent is menthol and/or eugenol or a mixture thereof. Such a composition may further comprise an anesthetic agent. Optionally, the disclosed composition further comprises 0.1% - 1.25% by weight of hyaluronic acid.

Advantageous amounts of components in a composition of the disclosure are:
0.005 - 0.05% by weight capsaicin,
0.025 - 0.3% by weight Cremophor® RH 40,
0.01 - 0.1% by weight phenol,
0.01 - 0.1% by weight menthol, and
0.2 - 1% by weight hyaluronic acid.

In one disclosure, the aqueous composition comprises
i) a capsaicinoid concentrate formed with a nonionic surfactant,
ii) an analgesic agent, and
iii) an aqueous vehicle to solubilize the capsaicinoid and analgesic agent in water.

The invention includes an optically clear solution containing aqueous and lipophilic ingredients which are totally miscible. The clear solution formed as a result of producing clear and stable aqueous mixtures, is a solution, a micelle, a microemulsion and a submicron emulsion or a suspension (i.e., of nano-sized particulates).

The invention also relates to compositions as claimed for use in methods for treating pain in a mammal; i.e., pain from osteoarthritis, rheumatoid arthritis, tendonitis, bursitis, a wound or surgical site, comprising:
i) administering a therapeutically effective amount of a corticosteroid to the site of pain; and subsequently
ii) administering a therapeutically effective amount of capsaicinoid composition to the site of pain.

Included are compositions as claimed for use in a method for treating joint pain in a mammal comprising:
i) administering a therapeutically effective amount of corticosteroid intra-articular to the joint; and subsequently
ii) administering a therapeutically effective amount of an aqueous capsaicinoid composition as claimed intra-articular to the joint.

Optionally, during the first minute after administering said capsaicinoid composition, the joint is repeatedly flexed and extended. An ice pack or cooling pack can be applied to the site, prior to, during, or after the capsaicinoid administration. In another embodiment of the compositions of the invention for use in treating joint pain, step i) is removed, i.e. the corticosteroid administration is not included.

Optionally, an anesthetic agent is administered intra-articular prior to, or simultaneously with said capsaicinoid composition. Examples are lidocaine, bupivacaine, ropivacaine, dibucaine, procaine, chloroprocaine, prilocaine, mepivacaine, etidocaine, tetracaine, and xylocaine, and mixtures thereof.

The invention also includes compositions of the present claims for use in a method for reducing the burning and stinging from administration of a capsaicinoid to a site in a mammal comprising administering a corticosteroid prior to administration of said capsaicinoid.

The corticosteroid is typically administered at least 4 hours prior to the administration of said capsaicinoid. Examples of the corticosteroid are dexamethasone acetate, methylprednisolone acetate, methylprednisolone sodium succinate, hydrocortisone acetate, and mixtures thereof. Advantageously, the costicosteroid selected is long acting.

In these methods, advantageously, the capsaicin dose level ranges from 0.001 mg to 1 mg., and the aqueous capsaicinoid composition is administered in a pharmaceutically acceptable vehicle in a volume from about 0.1 ml to 25 ml.

In one method disclosed herein, pain is treated by administration of a topical corticosteroid, advantageously a high potency corticosteroid topical preparation, prior to (30 minutes, 1 hour or 4 hours) topical administration of a topical capsaicinoid formulation- see US Application 2013/0157985 A1. In another disclosure, the topical corticosteroid is formulated with the capsaicinoid for topical application.

Also disclosed are kits for administering a capsaicinoid comprising:
a) at least one unit dose of a corticosteroid,
b) at least one unit dose of a capsaicinoid,
wherein the kit is arranged such that said glucocorticoid is administered prior to said capsaicinoid. The kit optionally includes at least one unit dose of an anesthetic agent (this is optionally formulated with the capsaicinoid), a means for administration of a) and b) (e.g. a syringe), instructions for administration, and/or a cooling means (e.g. a gel pack).

### Detailed Description of the Invention

The formulations of the invention provide a long-acting, non-opioid, treatment options for the management of moderate to severe chronic (greater than 3 months) or acute pain. Effective capsaicinoid injection therapies must include treatment modalities that address the potential intense acute burning pain following capsaicinoid administration.

Selective and reversible defunctionalization of sensory neurons in patients with disabling chronic pain conditions by site-specific capsaicin injections is an attractive approach for long lasting (weeks to months) pain relief. However, the treatment of joints etc. with the injection of capsaicin to relieve pain is complicated by the intense burning pain capsaicin elicits upon administration.

The capsaicinoid formulations and methods disclosed herein can be utilized to treat/attenuate pain in mammals typically via injection at a discrete site to provide pain relief for an extended period of time. The formulations are administered in a pharmaceutically acceptable vehicle for infiltration. The methods and formulations further include the administration of analgesic and/or anesthetic in an amount to attenuate the burning and hyperalgesia effects of capsaicinoid administration. This can be done in conjunction with the application of ice packs or gel packs, prior to or subsequent to capsaicinoid injection.

The present invention provides for administration of an aqueous capsaicinoid formulation for treatment of pain. A single dose of the capsaicinoid formulation is administered at a discrete site, a surgical site or open wound in an amount effective to denervate the injection site, surgical site (e.g. laparoscopy) or wound (e.g. bone fracture or torn ligament). Examples of sites are joints, muscles, tendons, nerves or tumors. Selective and reversible defunctionalization of sensory neurons by site-specific capsaicin injections provides long-lasting (weeks to months) pain relief in patients with disabling chronic pain conditions with no or minor systemic side effects.

The capsaicinoid formulations of the invention alleviate or attenuate pain at the site for a prolonged period of time. With respect to pain associated with arthritic conditions such as osteoarthritis, in certain embodiments, a single unit dose capsaicinoid injection or implantation attenuates pain at the site for at least about 3 months to at least about 4 months. With respect to joint pain, in certain embodiments, a single unit dose capsaicinoid injection or implantation attenuates pain at the site for at least about one month, at least about 3 months, and from about 3 to about 6 months. With respect to post-surgical pain, a single unit dose capsaicinoid injection or implantation attenuates pain at the site for at least about one week, and in certain embodiments for at least about 1 month.

### Compositions of the Invention

The dose of capsaicinoid is prepared for topical application, injection, implantation or infiltration by being incorporated into a pharmaceutically and physiologically acceptable aqueous vehicle for administration with diminished burning sensation upon application. The present invention is directed to the injectable administration of microgram and/or fractions of microgram quantities of capsaicin into discrete localized areas for the treatment and lessening of pain. Significant advantages result from milligram and/or fractions of milligram quantities of capsaicin in order to produce therapeutic results through alteration of sensory nerve function (TRPV-1) function in a limited area.

### Capsaicinoids

For a general discussion of capsaicinoids of the invention, see US patent application 2008/0262091, and commonly owned US Ser. No. 13/609,100 (***Pain Relief Compositions, Manufacture and Uses***)*.* The term "capsaicinoid" as used herein includes capsaicin, a capsaicinoid other that capsaicin, a mixture of capsaicin with one or more capsaicinoids. The amount of drug used being based on a therapeutically dose to a dose of capsaicin. Alternatively, a capsaicin analogue such as resiniferatoxin, can be administered in place of part or all of the capsaicinoid. The amount of analogue administered being the therapeutically equivalent dose of capsaicin-see US patent application 2008/0262091. In another embodiment, a TRPV1 agonist other than a capsaicinoid, is utilized in the formulations and methods of the invention.

### Delivery Vehicles

The aqueous pharmaceutical compositions of the invention utilize pharmaceutically acceptable delivery vehicles that are single phase aqueous/water systems composed of pharmaceutically acceptable solvents including polyethylene glycol (e.g. PEG 300 and PEG 400), ethanol and a nonionic surfactant, e.g. polysorbate 80 (PS 80); buffers; sodium chloride and/or sugar to control osmotic pressure gradients; and hyaluronic acid to control the viscosity of the formulation and aid in formulation stability. A significant advantage of the disclosed aqueous based formulations is that water concentrations can exceed 90 wt. %. Examples of additional components in the aqueous pharmaceutical vehicles include dextrose and/or sodium chloride solutions to adjust osmotic pressure and tonicity, as well as buffering agents to adjust pH. Further, the inclusion of 0.9% NaCl, 0.25% phenol, 0.25% menthol and 5% dextrose in the capsaicin/PS 80 aqueous solution did not result in a cloudy appearance or precipitate formation.

### Polyethylene Glycols

Polyethylene glycol, referred to as PEG, is used as an inactive ingredient in the pharmaceutical industry as a solvent, plasticizer, surfactant, ointment and suppository base, and in tablets and capsules as a lubricant. PEG has low systemic toxicity with systemic absorption less than 0.5%.The term "PEG" is used, in combination with a number. Within the pharmaceutical industry, the number indicates the mean molecular weight. The low-molecular weight liquid polyethylene glycols PEG 300 and 400 are excellent solvents and co-solvents for a large number of substances that do not readily dissolve in water. They are therefore widely used as solvents and solubilizing agents for active substances and excipients in liquid and semi-solid preparations. The ability of PEGs to form complexes with active substances is responsible for their excellent solvent power. Polyethylene glycols can also be used to adjust the viscosity of liquid pharmaceutical preparations and to modify their absorption properties and to stabilize the preparations.

Polyethylene glycols with a mean molecular weight up to 400 are non-volatile liquids at room temperature. Liquid PEGs up to PEG 600 are miscible with water in any ratio. But even higher molecular weight solid PEG grades have excellent solubility in water.

The polyethylene glycols show outstanding toxicological safety regarding acute and chronic oral toxicity, embryotoxicity or skin compatibility, supported by parenteral/ absorption/ excretion investigations. Therefore they have been used for many years in cosmetics, foodstuffs and the pharmaceutical industry. Many of these compounds are listed on the FDA Inactive Ingredient List for use in prescription products.

### Surfactants

A surfactant, such as a nonionic surfactant, e.g. PS 80 and/or Cremophor® RH 40 (Polyoxyl 40 Hydrogenated Castor Oil); alternatively, Cremophor® ELP, or Solute® HS 15), can be combined with one or more of the pharmaceutically acceptable vehicles previously described herein so that the surfactant agent serves as a wetting agent and emulsifier, and can lessen the initial stinging or burning discomfort associated with capsaicinoid administration.

Further, surfactant/capsaicin (or other capsaicinoids) concentrates can be formed for use in the formulations and methods of the invention as described in commonly owned U.S. Patent Application 2011/0311592 (***Methods of Increasing Solubility of Poorly Soluble Compounds and Methods of Making and Using Formulations of Such Compounds).***

### Alcohols

Additionally, alcohols including ethyl alcohol, glycerol, polyethylene glycols, etc. can be added to the formulations as effective capsaicin solubilizing agents and/or as safe for injectable.

### Hyaluronic Acid

The solubilization of capsaicin using the nonionic surfactants PS 80 and/or Cremophor® RH 40 together with hyaluronic acid and its salts thereof, a substance that is naturally present in the human body that occurs in various tissues (skin, synovial fluids of joints and connective tissues), contributes to ameliorating the burning and stinging associated with topical and injectable capsaicin formulations. Advantageously, ∼ 1% to 2% hyaluronic acid or its salts, is used. The hyaluronic acid molecular weight used in the experimental tests described herein had an average molecular weight ranging from 800 to 1,200 kDaltons. While not wishing to be bound by theory, it is believed that the addition of the hyaluronic acid component to the micellar solubilized capsaicin forms a polysaccharide network within the aqueous solution that stabilizes the micelles and serves to prevent micellar agglomeration and aggregation while causing capsaicin to be released more slowly in a controlled manner that results in a lessening of the burning and stinging pain.

### Analgesic Agents

Analgesic ingredients are used in the formulations of the invention to ameliorate or prevent the initial acute burning or stinging pain associated with capsaicin. A variety of analgesic agents can be used in the subject invention. Compositions of the invention comprise menthol and / or eugenol.

### Phenol and Menthol

Phenol and/or menthol can be administered at the surgical incision, open wound, or injection site to be treated. Phenol and menthol can be administered prior to administration of the capsaicinoid, or can be co-administered with the dose of capsaicinoid. The effective 24 hour intracapsular capsular concentration for bolus injected phenol and menthol concentrations should be ∼ 25 µg/ml or ∼ 250 µg for a joint fluid injection volume of 10 ml.

Both phenol and menthol (1) rapidly *penetrate* the surfaces in contact with the injected formulation; and (2) serve to *reduce or eliminate the acute burning and stinging pain sensation* associated with the administration of the TRPV1 agonist (e.g. capsaicin). Eugenol can also be used. The subject invention includes the use of specific injectable analgesics (phenol and menthol) that have a fast "onset of action" relative to capsaicin to effectively moderate the burning sensation effect of capsaicin. Onset of action of a compound is linked to its physicochemical properties; some agents are listed in Table III.

**TABLE III - Onset of Action of Selected Analgesic and Anesthetic Ingredients**

| **Ingredients** | **MW** | **Oil Soluble** | **Aqueous Soluble.** | **Log O/W** | **MP (°C)** | **Onset of Action** |
|---|---|---|---|---|---|---|
| **Capsaicin** | **305.41** | **soluble** | **insoluble** | **3.327** | **62-65** | **moderate** |
| **Ethyl Alcohol** | **46.07** | **soluble** | **miscible** | **-0.18** | **-114** | **fast** |
| **Phenol** | **94.11** | **soluble** | **soluble** | **10** | **43** | **fast** |
| **Menthol** | **156.26** | **soluble** | **Slightly soluble** | **2.66** | **42** | **fast** |
| **Lidocaine** | **234.34** | **soluble** | **insoluble** | **2.359** | **68** | **slow** |
| **Prilocaine** | **220.31** | **soluble** | **sparingly** | **2.11** | **137** | **slow** |
| **Benzocaine** | **165.19** | **soluble** | **sparingly** | **1.95** | **90** | **moderate** |

The use of these selected analgesics with a fast onset of action effectively moderates the burning effect of capsaicin when concomitantly administered, but also provides more immediate pain relief relative to capsaicin. In one embodiment of the invention, the injected analgesic agent has a molecular weight of 160 or less. Capsaicin provides more long term / long lasting pain relief relative to these fast onset of action injectable analgesics.

### Non-Steroidal Anti-Inflammatory Drugs (NSAIDs)

Non-steroidal anti-inflammatories, such as aspirin, naproxin, indomethacin, diclofenac sodium, refecoxib, and ibuprofen, inhibit the enzyme cyclooxygenase (COX-2 inhibitor) and therefore decrease prostaglandin synthesis. Prostaglandins are inflammatory mediators that are released during allergic and inflammatory processes. In whole, the NSAIDs prevent the prostaglandins from ever being synthesized, reducing or eliminating the pain.

An NSAID anti-inflammatory agent, such as diclofenac, aspirin, ibuprofen, naproxen, benoxaprofen, flurbiprofen, fenoprofen, and others, can be added to the injectable composition to provide localized pain relief with minimal or no systemic absorption.

### Anesthetic Agents

Local anesthetics such as lidocaine, are common additions to intra articular injections. They are used in many basic medical procedures to produce numbness for a short period of time, including being used in doctor's offices or at hospitals to numb an area that is injured or that requires minor surgical manipulation. A local anesthetic agent can be added to the injectable vehicle to provide localized pain relief. Examples of anesthetics are lidocaine, bupivacaine, ropivacaine, dibucaine, procaine, chloroprocaine, prilocaine, mepivacaine, etidocaine, tetracaine, and xylocale. Advantageously, the anesthetic is administered prior, ie 2-20 minutes, to the capsaicinoid administration due to differences in the onset of action of the compounds.

### Corticosteroids

In an advantageous embodiment of the invention, an adjunctive agent such as a corticosteroid (glucocorticoid) is administered prior to, or concurrently with, capsaicin to attenuate an initial acute burning and stinging pain from the administered dose of capsaicin. Some of the most potent anti-inflammatories are the corticosteroids. Corticosteroids have been shown to reduce inflammation by inhibiting the production of substances that cause inflammation. The use of injectable corticosteroids is widespread in pain management. These drugs can diminish or eliminate painful foci by virtue of their anti-inflammatory properties. Corticosteroid injection can be highly effective because it delivers the medication directly to the site of inflammation. The corticosteroid injection is an effective way to alleviate inflammation including that resulting from the inflammatory substances from the TRPV-1 nerve endings due to the presence of capsaicin. While not wishing to be bound by theory, it is believed that prior administration of the corticosteroids works by calming nerves and reducing the inflammatory effects of certain bio-transmitters, such as substance P and bradykinin, resulting from the subsequent capsaicinoid administration.

There are several corticosteroids that can be used including dexamethasone, methylprednisolone acetate, methylprednisolone sodium succinate and mixtures thereof. Other equivalent corticosteroids known to those skilled in the art can also be used. In one embodiment, the corticosteroid solubility is increased by the formation of a corticosteroid concentrate (e.g. using PS 80 or polyoxy 40 hydrogenated castor oil) according to US application 2011/0311592.

Injectable corticosteroids are available in either water-soluble or depot formulations. Water-soluble corticosteroids are typically not used for intra-articular injections because they rapidly diffuse from the injected area, exerting systemic effects. Depot formulations remain for a longer period of time at the injected site, maintaining a local effect, and are advantageous for joint injections. There are a variety of depot corticosteroids available for use including methyl prednisolone acetate, betamethasone sodium phosphate, betamethasone acetate, hydrocortisone acetate, prednisolone tebulate, triamcinolone acetonide, triamcinolone hexacetonide and mixtures thereof. Corticosteroids with lower solubility compared to compounds with greater solubility have an added benefit of maintaining effective synovial levels for a longer time and produce lower systemic levels.

When a local corticosteroid is administered prior to administration of the capsaicinoid, the corticosteroid administration provides burning and stinging pain relief to the area to be treated with the capsaicinoid (see Reference Example 6). The pre-administration of corticosteroids resulted in tolerable pain levels for the injection of a capsaicin containing formulations within intra-articular joint spaces.

Significantly, movement of the joint by bending the joint and/or walking within the first minute after capsaicin administration, served to circulate the formulation and reduce the burning and stinging pain. Cooling the joint via external cooling means such as gel and/or ice packs also reduces the painful effects from capsaicin administration.

The administration of the corticosteroid prior to the administration of capsaicin or capsaicin-like compounds results in less pain upon capsaicin administration, and pain relief at the site for a prolonged period of time - greater than 1 month, advantageously greater than 3 months, and most advantageously, greater than 6 months.

The use in orthopedic surgery of corticosteroids with their potent anti-inflammatory activity, with capsaicin formulations, eliminate or substantially reduce the acute pain from capsaicin administration. This allows extended pain relief in a safe and effective manner.

### Aqueous Based Capsaicin Injectable Formulations - Micellar/Free

The formulations cited in Table IV rely on the PEG and ethyl alcohol to solubilize capsaicin and the analgesic agents, phenol and menthol. A nominal content of ∼ 15 - 40 wt.% PEG and ∼ 0 - 40 wt.% ethyl alcohol are required to maintain a single-phase aqueous solution of phenol and menthol.

**TABLE IV - High PEG 300/400 Capsaicin Formulations (micellar free)**

| **INGREDIENT** | **FUNCTION** | **CONCENTRATION RANGE - (wt.%)** | **Preferred Conc. (wt. %)** |
|---|---|---|---|
| **Capsaicin** | **Defunctionalization of TRPV-1 sensory neurons** | **0.0002-0.1** | **∼ 0.01** |
| **PEG 300/400** | **Solubilizing agent** | **15 - 50** | **∼ 30** |
| **Hyaluronic Acid** | **Viscosity enhancer, stabilizing & moisturizing agent,** | **0.25 - 1.5 ( can vary the range of Molecular weights to achieve desired viscosity 800-1,500 kDaltons)** | **∼ 1.0 (∼ 1,000 kDaltons)** |
| **Phenol, USP** | **Analgesic/anesthetic and antiseptic agent** | **0 - 1** | **∼ 0.1** |
| **Eugenol, USP** | **Analgesic/anesthetic and antiseptic agent** | **0 - 1** | **∼ 0.1** |
| **Menthol, USP** | **TRPV-8 Cooling & analgesic agent** | **0 - 1** | **∼ 0.1** |
| **Sodium Chloride / Sucrose** | **Tonicity agents** | **NaCl - < 0.9 -** | **NaCl ∼0.9** |
| | | **Sugar ∼ 5** | **Sucrose ∼ 5** |
| **Water** | **Solvent (pyrogen free)** | **q.s.** | **q.s.** |
| **Phosphate Buffer /Citrate Buffer** | **pH Control** | **Adjust pH from 7.0 - 8.0** | **∼** 7.2 |
| **Diclofenac Sodium (a NSAID)- optional** | **Anti-Inflammatory agent** | **0 - 1** | **∼ < 1** |

### Aqueous Based Capsaicin Injectable Formulations - Containing Capsaicin in Micelles

As noted in Examples V and Examples VI A to VI D of patent application US 2006/0148903, compositions containing 1mg/ml of capsaicin within an aqueous solution required 10 mg/ml of PS80 in aqueous solutions. It was also noted that for each 10 mg/ml increase in the PS 80 concentration that the aqueous solubility of capsaicin increases by about 1 mg/ml.

The examples of commonly owned U.S. Patent Application 2011/0311592 ***(Methods of Increasing Solubility of Poorly Soluble Compounds and Methods of Making and Using Formulations of Such Compounds)*** however, unexpectedly teach requiring one-half (½) the PS 80 concentration within an aqueous solution; i.e., 5 mg/ml of PS 80 to solubilize 1mg/ml of capsaicin. The proportional 1 mg/ml concentration in the relatively aqueous insoluble capsaicin achieved with 5 mg/ml of PS 80 indicates that capsaicin is contained within micelles. (See Reference Example 1) Therefore, to achieve a capsaicin concentration of 0.06 mg/ml, only 0.3 gm/ml of PS 80 is required.

The formulations cited in Table V below rely on the PS 80 to solubilize capsaicin via micellar formation and the PEG and ethyl alcohol content to solubilize the analgesic agents, phenol and menthol. A 5 - 20 wt. % of PEG and 0 - 40 wt.% ethyl alcohol are required to maintain a single-phase aqueous solution of phenol and menthol.

**TABLE V - PS 80 Capsaicin Formulations**

| **INGREDIENT** | **FUNCTION** | **CONCENTRATION RANGE - (wt.%)** | **Preferred Cone. (wt. %)** |
|---|---|---|---|
| **Capsaicin** | **Defunctionalization of TRPV-1 sensory neurons** | **0.0002-0.1** | ∼ **0.01** |
| **Polysorbate 80** | **Capsaicin Solubilizing via micelles** | **0.001** - **10** | **∼ 0.5** |
| **PEG 300/400** | **Solubilizing agent** | **5 - 20** | ∼ **15** |
| **Hyaluronic Acid** | **Viscosity enhancer, stabilizing & moisturizing agent,** | **0.25 - 1.5 (can vary the range of Molecular weights to achieve desired viscosity 800-1,500 kDaltons)** | **∼ 1.0 (∼ 1,000 kDaltons)** |
| **Phenol, USP** | **Analgesic/anesthetic and antiseptic agent** | **0 - 1** | **∼ 0.1** |
| **Eugenol, USP** | **Analgesic/anesthetic and antiseptic agent** | **0-1** | ∼ **0.1** |
| **Menthol, USP** | **TRPV-8 Cooling & analgesic agent** | **0** - **1** | **∼ 0.1** |
| **Sodium Chloride / Sucrose** | **Tonicity agents** | **NaCl - < 0.9** | **NaCl ∼ 0.9** |
| | | **Sugar ∼ 5** | **Sucrose ∼ 5** |
| **Water** | **Solvent (pyrogen free)** | **q.s.** | **q.s.** |
| **Phosphate Buffer /Citrate Buffer** | **pH Control** | **Adjust pH from 7.0** - **8**.**0** | ∼ **7.2** |
| **Diclofenac Sodium (a NSAID)- optional** | **Anti-inflammatory agent** | **0 - 1** | **∼ < 1** |

The formulations cited in Table VI below rely on Cremophor® RH 40 (alternatively, Cremophor® ELP, or Solute® HS 15) to solubilize capsaicin and the analgesic agents, phenol and menthol.

**TABLE VI - Cremophor® RH 40 Capsaicin Formulations**

| **INGREDIENT** | **FUNCTION** | **CONCENTRATION RANGE - (wt.%)** | **PREFERRED CONC. (wt.%)** |
|---|---|---|---|
| Ca**psaicin** | **Defunctionalization of TRPV-1 sensory neurons** | **0.0002 - 0.1** | **∼0.01** |
| **Cremophor® RH 40 or Cremophor® ELP, or Solute® HS 15** | **Solubilizing Complexing Agents via micelle formation** | **0.001-1.0** | **∼0.06** |
| **Hyaluronic Acid** | **Viscosity enhancer, Stabilizing and moisturizing agent** | **0.25 - 1.5 (can vary the range of Molecular weights to achieve desired viscosity preferably 800 -1,500 kDaltons)** | **∼ 1.0 (∼ 1,000 kDaltons)** |
| **Phenol, USP** | **Analgesic/anesthetic and antiseptic agent** | **0-1** | **∼ 0.1** |
| **Menthol, USP** | **Analgesic/anesthetic and antiseptic agent** | **0-1** | **∼ 0.1** |
| **Eugenol, USP** | **Analgesic/anesthetic and antiseptic agent** | **0-1** | **∼ 0.1** |
| **Sodium Chloride / Sucrose** | **Tonicity agents** | **NaCl - < 0.9 -** | **NaCl - ∼ 0.9** |
| | | **Sucrose ∼ 5** | **Sucrose - ∼ 5** |
| **Water** | **Solvent (pyrogen free)** | **q.s.** | q.s. |
| **Phosphate Buffer/Citrate Buffer** | **pH control** | **Adjust pH from 7.0 - 8.0** | **∼ 7.2** |
| **Diclofenac Sodium (a NSAID) - optional** | **Anti-inflammatory agent** | **0-1** | **∼ < 1** |

### Gels

The addition of polyethylene glycols, such as PEG-300 or PEG-400, (∼5 -10%) to oil-based liquid formulations allows for the addition of water (∼10%). Subsequent gels have been formed with the addition of cellulosic gelling additives.

### Uses and Administration of the Compositions of the Invention

The formulations of the present invention are useful in 1) relieving pain at an intra-articular site or at a body space; 2) alleviating the post surgical pain experienced by patients following discharge from a clinical care facility; 3) providing effective post-surgical analgesia such that the amount of narcotics taken by a patient is reduced thereby decreasing post-surgical rehabilitation time.

Uses and methods and of the compositions of the inventions include but are not limited to orthopedic disorders of the knee, shoulder, hip, back, spine, neck, elbows, hand, foot and other disorders which involve pain at a specific site. Examples of intra-articular administration include injection to the knee, elbow, hip, carpal, tarsal, wrist, intervertebral disk, ankle, and any other joints subject to arthritic conditions. Examples of body spaces include bursae or peritoneum.

The compositions of the invention can be used for management and prolonged relief of nociceptive pain in mammals associated with osteoarthritis, rheumatoid arthritis, tendonitis, bursitis, pre- and post-surgical conditions, as well as for neuropathic pain occurring secondary to nerve injury. Other uses and methods of administration are described in US patent 8,420,600, and commonly owned US Ser. No. 13/609,100 (*Pain Relief Compositions, Manufacture and Uses*)*.*

As used herein, "injection" means administration to a site through the skin. "Implantation" shall mean administration at a site by embedding a dose of material into the skin, muscle, tendon or joint.

The capsaicin formulations can be administered via injection or infiltration to a site. For surgery or wounds, the dose is administered to the muscle, tissue or bones surrounding the surgical or wound site. When the capsaicinoid is administered by infiltration, the capsaicinoid is administered to the surgical site or wound with an instrument known to those skilled in the art for administering via infiltration, e.g. a needle and syringe.

As used herein, "therapeutically effective amount" refers to that quantity or dose of an agent to produce a clinically desired result such as a biological response, or a reduction of a symptom of a disease or condition, e.g. reduction in or elimination of pain.

When the single dose of capsaicin is administered via injection, the injection volume of capsaicin depends on the localized site of administration. Suitable injection volumes to be delivered advantageously range from about 0.1 to about 20 ml, more advantageously from about 0.5 to about 10 ml and most advantageously from about 1.0 to about 5 ml, depending on the site to be treated.

Advantageous use of the injectable compositions of the invention for treatment of joint pain and to minimize or eliminate the burning and stinging effects of injectable capsaicin follows. Sterile technique must be used for injections in order to reduce the risk of infection. The skin is initially cleaned with Betadine or other suitable antiseptic agent.
- A needle is inserted into the joint and excess fluid is withdrawn.
- A corticosteroid (advantageously slow acting) fluid is injected into the intraarticular region. It is recommended to allow this corticosteroid injection to infiltrate and penetrate the surrounding tissue for a period of time necessary to diminish or eliminate the burning and stinging that will occur upon capsaicinoid injection (greater than 1 hour, advantageously greater than 4, 8, 16 or 24 hours).
- The corticosteroid injection is optionally followed by administration of an anesthetic agent such as Lidocaine to further anesthetize the intra articular region. After ∼ 5 - 10 minutes (to allow the anesthetic agent to infiltrate the surrounding intra articular surfaces), the capsaicinoid formulation is administered. Advantageously, the anesthetic agent is not removed from the joint prior to administration of the capsaicinoid.
- In an advantageous embodiment, the joint is worked (flexed and extended) repeatedly for the first 1 minute (advantageously 30 seconds) after administration of the capsaicinoid. An ice pack or cooling pack is optionally applied to the joint before, during and/or after the joint is flexed and extended.

The methods of the invention use trans-capsaicin dose level ranges from 0.001 mg to 1.0 mg., advantageously from 0.2 mg. to 0.4 mg. The methods utilize an aqueous vehicle in a volume from about 0.1 ml to 25 ml, advantageously 5-10 ml. The amounts used of capsaicinoid and vehicle for small joint or non-joint applications will vary as determined by a person skilled in the art.

The formulations and methods of the subject invention provide pain relief for 2 to 5 days, advantageously 7 to 21 days, or more advantageously 6 to 8 weeks, or greater than 14 weeks (1 to 4 months).

Similar techniques can be used for the relief of pain relief other than joint pain.

The following examples are illustrative, but not limiting of the compositions and methods of the present invention. Other suitable modifications and adaptations of a variety of conditions and parameters normally encountered that are obvious to those skilled in the art are within the spirit and scope of the invention.

### REFERENCE EXAMPLE 1

### Preparation of a Concentrated Capsaicin/PS 80 Solution

U.S. Patent Application 2011/0311592 **(*****Methods o**f* ***Increasing Solubility ofPoorly Soluble Compounds and Methods of Making and Using Formulations of Such Compounds*)** teaches 5 mg/ml of PS 80 are required to solubilize 1mg/ml of capsaicin; i.e., to achieve a capsaicin concentration of 0.06 mg/ml, only 0.3 gm/ml of PS 80 are required. Also, it was determined that the relatively aqueous insoluble capsaicin is contained within micelles since for each 1 mg/ml increase in the capsaicin solubility, 5 mg/ml of PS 80 was required. The teachings of this application were utilized in solubilizing capsaicin in a high purity PS 80 obtained from Croda Inc.

### STEP I - THE PREPARATION OF A CAPSACIN and PS 80 CONCENTRATE

### Ingredients Include:

- 10 grams of Polysorbate 80 (PS 80), Super refined, Croda Inc., CAS # 9005-65-6
- 2 grams of Trans-Capsaicin, Aversion Technologies Inc., USP 30, 95.7% Trans-Capsaicin, Balance Cis-Capsaicin

### Procedure:

**1.** 10 grams of PS 80 are added to a 50 ml "Pyrex" glass vial.
**2.** 2 grams of Trans-Capsaicin are added to the PS 80 in Step 1.
**3.** The mixture from Step 2 is heated to ∼ 55 °C to dissolve the Trans-Capsaicin and form the Trans-Capsaicin/PS80 concentrate.

The addition of a few capsaicin particulates to the room temperature Capsaicin/PS 80 solution did not result in capsaicin precipitates being formed thus indicating that the capsaicin solution was not supersaturated. Unexpectedly high concentrations levels were achieved; i.e., 200 mg capsaicin in 1.0 ml of PS 80 (200 mg/ml). The addition of 2 ml of this capsaicin/PS 80 concentrate to 98 grams of∼ 70 °C water resulted in a cloudy solution which gradually became crystal clear as the solution mixture cooled to room temperature. Thus the addition of this 20% capsaicin/PS 80 concentrate to water resulted in an aqueous capsaicin solubility level of 4 mg/ml. Similarly, the addition of 10 ml of this capsaicin/PS80 concentrate to 90 grams of∼ 70 °C water resulted in a cloudy solution which gradually became crystal clear as the solution mixture cooled to room temperature. Thus, the addition of this 2% capsaicin/PS 80 concentrate to water resulted in an aqueous capsaicin solubility level of 20 mg/ml.

It was also noted that for each 5 mg/ml increase in the PS 80 concentration that the aqueous solubility of capsaicin increases by about 1 mg/ml. While not wishing to be bound by theory, this suggests that the capsaicin is contained in PS 80 micelles.

### REFERENCE EXAMPLE 2

### Preparation of a 200 gram Aqueous Solution Containing the Capsaicin/PS80 Concentrate from Reference Example 1 and Hyaluronic Acid

### STEP I - THE PREPARATION OF THE HYALURONIC ACID CONCENTRATE

### Ingredients Include:

- 1 gram of Hyaluronic Acid, M.W. = 1,000 kDaltons, Lotioncrafter LLC, CAS # 9067-32-7
- 191.8 gram of Distilled Water, Poland Springs

### Procedure:

**1.** Add 1 gram of Hyaluronic Acid in a 400 cc Pyrex beaker.
**2.** Add 191.8 grams of water to the Hyaluronic Acid and thoroughly mix until a clear solution is obtained. It takes an extended time duration to completely solubilized the hyaluronic acid; mostly likely 30 minutes.

### STEP II - THE PREPARATION OF THE 0.6 mg/ml Capsaicin, 3.0 mg/ml PS80 & 5 mg/ml Hyaluronic Acid Aqueous Solution

### Ingredients Include:

- 7.2 grams of the Capsaicin/PS 80 concentrate from Reference Example 1.
- 192.8 grams of the Hyaluronic Acid from STEP I.

### Procedure:

**1.** To the 192.8 grams of the Hyaluronic Acid Solution prepared in STEP I and contained within a 400 cc Pyrex beaker, slowly add 7.2 grams of the Capsaicin/PS 80 prepared in Reference Example 1 while thoroughly stirring.
**2.** The mixture from Step 1 is now ready for subsequent packaging.

After thoroughly mixing the ingredients, the resulting mixture was crystal clear and was moderately viscous. The viscosity of the mixture can be adjusted by varying the hyaluronic acid content.

The elevated temperature solubilization of capsaicin in PS 80 is important. Several attempts to add the PS 80 and capsaicin to water followed by heating to temperatures of ∼ 70 °C - 90 °C resulted in only a fraction of the capsaicin dissolving.

The ability to achieve increased capsaicin solubility levels within an aqueous medium as described in the foregoing text, allows for significant reductions in the liquid volume of capsaicin to be injected. Experiments with PEG-400 did not achieve the capsaicin solubility levels when compared to the PS80 (micellar solubilization) surfactant.

Experiments also indicate the formation of capsaicin containing gels with the addition of gelling agents such as HPMC, HEC. Carbomers, etc. have been formed.

### EXAMPLE 3

### Preparation of 200 grams of a 0.06 mg/ml Capsaicin Injectable Solution (micelle free)

### STEP I - THE PREPARATION OF A CAPSACIN, PHENOL, MENTHOL SOLUTION

### Ingredients Include:

- 60 grams of PEG-300, Spectrum Chemical, NF, CAS # 25322-68-3
- 16 grams of Ethyl Alcohol, Graves Grain Alcohol, 190 Proof
- 0.2 grams of Phenol, Liquefied (carbolic Acid), Spectrum Chemical, USP, CAS # 108-95-2
- 0.2 grams of L-Menthol, Crystal, Spectrum Chemical, USP, CAS # 2216-51-5
- 0.012 grams of Trans-Capsaicin, Aversion Technologies Inc., USP 30, 95.7% Trans-Capsaicin, Balance Cis-Capsaicin

### Procedure:

**1.** Add 60 grams of PEG-300 in a 400 cc Pyrex beaker.
**2.** Add 16 grams of Ethyl Alcohol to the PEG-300 & thoroughly mix.
**3.** Add 0.2 grams of Liquefied Phenol to the mixture of Step 2.
**4.** Add 0.2 grams of L-Menthol Crystals to the mixture of Step 3.
**5.** Add 0.012 grams of Trans-Capsaicin to the mixture of Step 4
**6.** Heat the mixture of Step 5 to ∼ 40 °C to hasten the formation of the solution of menthol and capsaicin.
**7.** The solution from Step 6 is set aside and allowed to cool to room temperature.

### STEP II - THE PREPARATION OF THE HYALURONIC ACID SOLUTION

### Ingredients Include:

- 1 gram of Hyaluronic Acid, M.W. = 1,000 kDaltons, Lotioncrafter LLC, CAS # 9067-32-7
- 122.6 gram of Distilled Water, Poland Springs

### Procedure:

**1.** Add 1 gram of Hyaluronic acid in a 250 cc Pyrex beaker.
**2.** Add 122.6 grams of water to the Hyaluronic Acid and thoroughly mix until a clear solution is obtained. It takes an extended time duration to completely solubilized the hyaluronic acid; mostly likely 30 minutes.

### STEP III - THE COMBINING OF STEP I & STEP II SOLUTIONS

### Ingredients Include:

- The solution mixture from STEP I
- The solution mixture from STEP II.

### Procedure:

**1.** The hyaluronic acid solution from STEP II is slowly added to the capsaicin containing solution from Step I while thoroughly stirring.
**2.** The mixture from Step 1 is now ready for subsequent packaging.

Table 6 contains the ingredients contained within the 0.06 mg/ml capsaicin solution.

### EXAMPLE 4

### Preparation of 200 grams of a 0.06 mg/ml Capsaicin/PS 80 Micelle Injectable Solution

### STEP I - THE PREPARATION OF A CAPSACIN and PS 80 CONCENTRATE

### Ingredients Include: (Note: To ensure weighing accuracy, the amount of the Capsaicin/PS 80 concentrate prepared was 8 times that required for a 0.06 mg/ml injectable solution)

- 2 grams of Polysorbate 80 (PS 80), Super refined, Croda Inc., CAS # 9005-65-6
- 0.125 grams of Trans-Capsaicin, Aversion Technologies Inc., USP 30, 95.7% Trans-Capsaicin, Balance Cis-Capsaicin

### Procedure:

**1.** 2 grams of PS 80 are added to a 16 ml Pyrex glass vial.
**2.** 0.125 grams of Trans-Capsaicin are added to the PS 80 in Step 1.
**3.** The mixture from Step 2 is heated to ∼ 55 °C to dissolve the Trans-Capsaicin and form the Trans-Capsaicin/PS 80 concentrate.

### STEP II - THE PREPARATION OF THE CAPSAICIN/PS 80, PEG-300, ETHYL ALCOHOL, PHENOL & MENTHOL SOLUTION

### Ingredients Include:

- 20 mg of PEG PEG-300, Spectrum Chemical, NF, CAS # 25322-68-3
- 10 grams of Ethyl Alcohol, Graves Grain Alcohol, 190 Proof
- 0.27 grams of the Trans-Capsaicin/PS 80 concentrate from STEP I.
- 0.2 grams of Phenol, Liquefied (carbolic acid), Spectrum Chemical, USP, CAS # 108-95-2
- 0.2 grams of L-Menthol, Crystal, Spectrum Chemical, USP, CAS # 2216-51-5

### Procedure:

**1.** Add 20 grams of PEG-300 in a 400 cc Pyrex beaker.
**2.** Add 10 grams of Ethyl Alcohol to the PEG-300 and thoroughly mix.
**3.** Add 0.27 grams of the Trans-Capsaicin/PS 80 concentrate to the mixture from Step 2 and thoroughly stir.
**4.** Add 0.2 grams of Liquefied Phenol to the mixture of Step 3.
**5.** Add 0.2 grams of L-Menthol Crystals to the mixture of Step 4.
**6.** Heat the mixture from Step 5 to ∼ 40 °C to hasten the solution of menthol.
**7.** The solution from Step 5 is set aside and allowed to cool to room temperature

### STEP III - THE PREPARATION OF THE HYALURONIC ACID SOLUTION

### Ingredients Include:

- 1 gram of Hyaluronic Acid, M.W. = 1,000 kDaltons, Lotioncrafter LLC, CAS # 9067-32-7
- 169.5 grams of Distilled Water, Poland Springs

### Procedure:

**1.** Add 1 gram of Hyaluronic acid in a 400 cc Pyrex beaker.
**2.** Add 169.5 grams of water to the Hyaluronic Acid and thoroughly mix until a clear solution is obtained. It takes an extended time duration to completely solubilized the hyaluronic acid; mostly likely 30 minutes.

### STEP III - THE COMBINING OF STEP I & STEP II SOLUTIONS

### Ingredients Include:

- The solution mixture from STEP I
- The solution mixture from STEP II.

### Procedure:

**1.** The hyaluronic acid solution from STEP II is slowly added to the capsaicin containing solution from STEP II while thoroughly stirring.
**2.** The mixture from Step 1 is now ready for subsequent packaging.

Table 6 contains the ingredients contained within the 0.06 mg/ml capsaicin solution.

### REFERENCE EXAMPLE 5

### Preparation of 200 grams of a 4mg/ml Capsaicin, 1mg/ml Menthol and 1mg/ml Phenol Transparent Aqueous Concentrate

The following steps were followed in the preparation of a 4 mg/ml Capsaicin, 1 mg/ml Menthol and 1 mg/ml Phenol concentrate.

### Ingredients Include:

- 0.40 grams of the Trans-Capsaicin, Aversion Technologies Inc., USP 30, 95.7% Trans-Capsaicin, Balance Cis-Capsaicin
- 0.10 grams of Phenol, Liquefied (carbolic acid), Spectrum Chemical, USP, CAS # 108-95-2
- 0.10 grams of L-Menthol, Crystal, Spectrum Chemical, USP, CAS # 2216-51
- Cremophor® RH 40, CAS Number 61788-85-0 obtained from Sigma Aldrich, St. Louis, MO

### Procedure:

**1.** Add 0.4 grams of capsaicin powder, 0.1 grams of menthol crystals and 0.1 grams of liquefied phenol to a 300 cc Pyrex beaker.
**2.** Add 2.0 grams of Cremophor® RH 40 to the mixture from Step 1.
**3.** The mixture from Step 2 is heated to about 125 °C.
**4.** Water is slowly added to the mixture from Step 3 while thoroughly stirring to provide an aqueous mixture weighing 200 grams.

An optically clear aqueous solution containing 4 mg/ml Capsaicin, 1 mg/ml Menthol, 1 mg/ml Phenol and 20 mg/ml Cremophor® RH 40 resulted. A combined weight ratio of the capsaicin, menthol and phenol mixture to the Cremophor® 40 surfactant was 1.0/3.33.

### REFERENCE EXAMPLE 6

### Effect of Corticosteroid Pretreatment on Pain Due to Capsaicinoid Injection in Humans

This study examined the administration of a corticosteroid as an adjunctive agent prior to the administration of capsaicin to attenuate the burning and stinging effect resulting for the intra-articular injection of capsaicin into the knee joint.

### THE CAPSAICIN FORMULATION

Johnson Compounding of Waltham, MA supplied the injection formulation as a sterile liquid capsaicin solution and a sterile dilution vehicle for diluting the capsaicin solution prior to use for injection.

Both the capsaicin solution and dilution vehicle were manufactured and released in compliance with GMPs. Lot release testing included tests for Appearance, Potency, Purity (HPLC), Impurities (HPLC), Bacterial Endotoxin, and Sterility (USP<71>).

The capsaicin solution was manufactured by dissolving capsaicin in 100% polyethylene glycol 300 (PEG 300). The resulting solution was sterile filtered and aseptically filled into 5 ml sterile Type I glass vials which are then sealed. The components and compositions of the capsaicin solution are listed in Table VI1.

**TABLE VI1 - Components of the Capsaicin Solution**

| **INGREDIENTS** | **CONCENTRATION** | **FUNCTION** |
|---|---|---|
| **^{*(1*)} 99+ wt.% Trans- Capsaicin** | **0.04 wt. % (0.4 mg/ml)** | **API** |
| ***⁽²⁾* PEG-300 Ph Eur** | **Balance** | **Solvent** |

| | | |
|---|---|---|
| ***⁽¹⁾ The cGMP 99⁺ wt. % Capsaicin procured from Formosa Laboratories, Taiwan*** ***⁽²⁾ Sigma-Aldrich, St. Louis MO, (Catalog*** # ***81162)*** | | |

The components and compositions of the pharmaceutical vehicle utilized to deliver the capsaicin are listed in Table VIII.

**TABLE VIII - Components of Capsaicin Vehicle**

| **INGREDIENTS** | **CONCENTRATION** | **FUNCTION** |
|---|---|---|
| *⁽¹⁾* **PEG-300 Ph Eur** | **20 wt.%** | **Solvent** |
| *⁽²⁾* **L- Histidine** | **0.18 wt.%** | **Buffering agent** |
| *⁽³⁾* **Sucrose** | **6 wt.%** | **Isotonic agent** |
| **Water** | **Balance** | **Solvent** |

| | | |
|---|---|---|
| ***⁽¹⁾ Sigma-Aldrich, St. Louis MO, (Catalog # 81162)*** ***⁽²⁾ Sigma-Aldrich, St. Louis MO, (Catalog #H3911)*** ***⁽³⁾ Sigma-Aldrich, St. Louis MO, (Catalog # 84097)*** | | |

### CLINICAL STUDY

Tests were conducted to evaluate the effectiveness of a corticosteroid injection into a site prior to injection of a capsaicin formulation. Subjects with arthritic knee joints were selected and treated as follows:
- The knee joint to be treated was cleaned with Betadine or other suitable antiseptic agent.
- A needle was inserted into the joint and excess fluid withdrawn.
- A dose level of 60 mg of Depo-Medrol® (methylprednisolone acetate injectable suspension, USP) was injected in the treated joints a day prior to the administration of the capsaicin injection. Depo-Medrol® is an anti-inflammatory glucocorticoid for intra-articular, soft tissue, intramuscular or intralesional injection.
- On the day following the corticosteroid injection, an anesthetizing agent (1 % Lidocaine) was injected into the joint and allowed to infiltrate the surrounding intra-articular surfaces for ∼ 5 minutes.
- Capsaicin at dose level of 0.2 mg in 5 ml was then injected into the joint (within 30 minutes of the Lidocaine injection).

### RESULTS

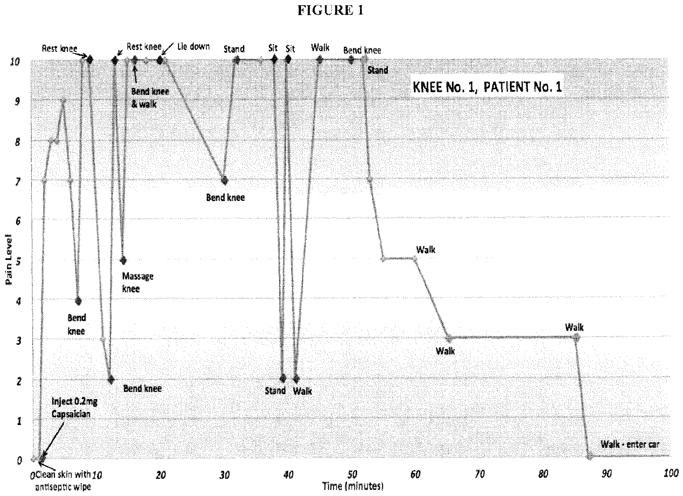

Procedures and resulting pain level experienced by the injection of subjects injected with a 0.2 mg dose level of the capsaicin formulation as a function of elapsed time are summarized in Tables IX to XV. These results are graphically illustrated in Figures 1 to 6. A description of the 6 knees treated with the capsaicin injections follows.

### INJECTED KNEE No. 1, PATIENT No.1

A 76 year old male with painful osteoarthritis of both knees received a 60 mg dose of Depo-Medrol (methylprednisolone acetate) injected into intra-articular cavity of his left knee. On the following day, the knee was cleansed with an antiseptic pad one minute prior to the injection of a 5 ml solution containing 0.2 mg capsaicin. Following the capsaicin injection, the subject experienced extreme pain for a duration of about 1 hour before the pain level subsided to tolerable levels. Bending and straightening and multiple applications of ice packs to the joint did not result in alleviating the discomfort from the capsaicin injection. The procedure required nearly 80 minutes before the subject encountered significant relief of his arthritic pain symptoms. TABLE IX and Figure 1 include summaries of procedural pain levels as a function of elapsed time. This is the only knee in this Example that did not receive Lidocaine.

**TABLE IX**

| **INJECTED KNEE # 1 (Patient # 1, left knee treated)** | | |
|---|---|---|
| **ELAPSED TIME (Minutes)** | **PROCEDURE** | **PAIN SCORE (0 - 10)** |
| **0** | **Clean skin with an antiseptic wipe** | **0** |
| **1** | **Inject 0.2 mg Capsaicin** | **tinglling** |
| **2** | --- | **7** |
| **3** | --- | **8** |
| **4** | --- | **face hot,** |
| **5** | **---** | **9** |
| **6** | **---** | **7** |
| **7** | **---** | **4** |
| **8** | **Bend knee** | **10** |
| **9** | **---** | **deep pain** |
| **11** | **---** | **3** |
| **12** | --- | **2** |
| **13** | **Bend knee** | **10** |
| **14** | **Rest knee** | **5** |
| **15** | **Massage knee** | **10** |
| **16** | **Bend knee** | **10** |
| **18** | **Walk** | **10** |
| **20** | **Lie down** | **10** |
| **21** | --- | **10** |
| **22** | **Iee pack to knee** | **10** |
| **24** | **Bend knee** | **9** |
| **25** | **Rest** | **3** |
| **29** | **Walk** | **8** |
| **30** | **Lie down** | **7** |
| **32** | **Bend knee** | **10** |
| **36** | **Stand** | **10** |
| **38** | **Weight on leg** | **10** |
| **39** | **Sit** | **2** |
| **40** | **Weight on leg** | **10** |
| **41** | **Sit** | **2** |
| **45** | **Walk** | **10** |
| **48** | **Bend** | **10** |
| **55** | **Stand** | **5** |
| **60** | **Stand** & **walk** | **5** |
| **80** | **Walking** | **3** |
| **82** | **Get in car** | **0** |

### INJECTED KNEE No. 2, PATIENT No. 2

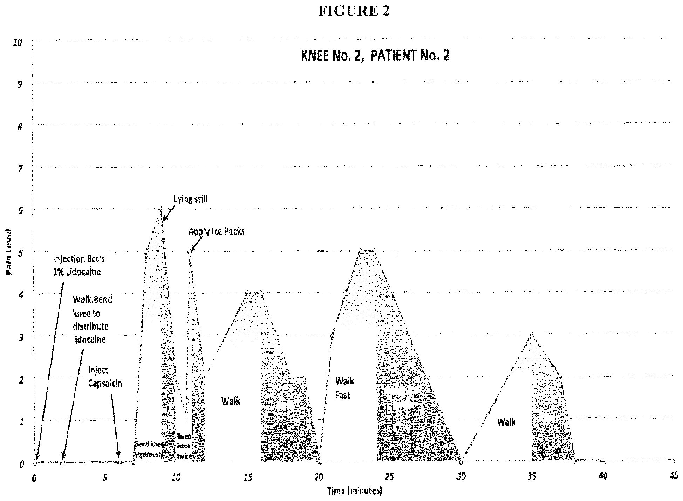

A 67 year old male with painful osteoarthritis of both knees received a 60 mg dose of Depo-Medrol (methylprednisolone acetate) injected into the intra-articular cavity of his left knee. On the following day, 8 ml of a solution containing 1% Lidocaine was injected into the intra-articular cavity of this same knee and distributed throughout the joint cavity by walking and bending of the knee for about 4 minutes prior to intra-articular injection of a 5 ml solution containing 0.2 mg capsaicin. Immediately after the capsaicin injection the knee was bent and straightened vigorously for ∼ 30 seconds to distribute the capsaicin solution within the joint cavity. The pain level briefly rose to a moderate level and quickly subsided upon resting and applying ice packs to this knee. After about 30 minutes, the procedure was completed and the subject experienced complete relief of his osteoarthritic pain symptoms. TABLE X and Figure 2 include summaries of procedural pain levels as a function of elapsed time.

**TABLE X**

| **INJECTED KNEE # 2 (Patient #2, left knee treated)** | | |
|---|---|---|
| **ELAPSED TIME (Minutes)** | **PROCEDURE** | **PAIN SCOR E (0 - 10)** |
| **0** | **Inject 8 cc 1% Lidocaine** | **0** |
| **2** | **Walk & bend knee to distribute Lidocaine** | **0** |
| **6** | **Inject 0.2 mg Capsaicin** | **0** |
| **7** | **Bend knee vigorously** | **0** |
| **8** | **Pain** | **5** |
| **9** | **Lying still** | **6** |
| **10** | **Bend knee twice** | **2** |
| **11** | **-----** | **1** |
| **11** | **Bend knee** | **5** |
| **11** | **Apply 2 ice packs** | **5** |
| **12** | **Rest** | **2** |
| **15** | **Walk** | **4** |
| **16** | **-----** | **4** |
| **17** | **Rest** | **3** |
| **18** | **------** | **2** |
| **19** | **Rest - face flushed** | **2** |
| **20** | **Walk fast** | **0** |
| **21** | **Walk fast** | **3** |
| **22** | **Walk fast** | **4** |
| **23** | **Walk fast** | **5** |
| **24** | **Ice applied** | **5** |
| **30** | **Ice applied** | **0** |
| **30** | **Walk** | **0** |
| **35** | **Walk** | **3** |
| **37** | **Sit** | **2** |
| **38** | **Sit** | **0** |
| **40** | | **0** |

### INJECTED KNEE No. 3, PATIENT No. 3

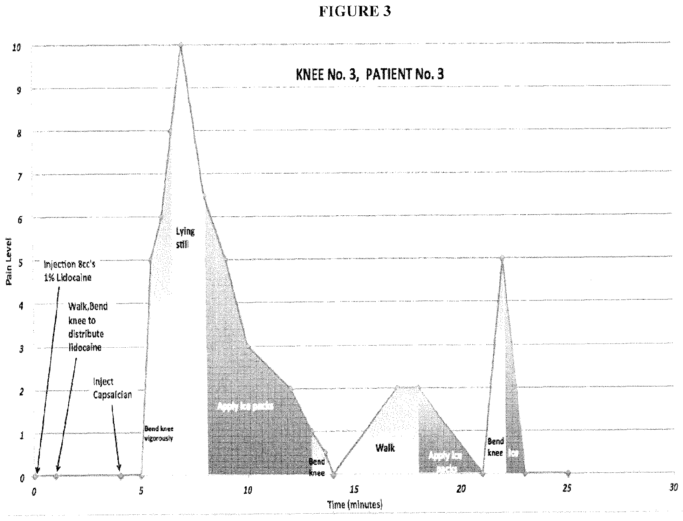

A 67 year old male who was previously diagnosed with severe and painful osteoarthritis of both knees and scheduled for a left knee replacement in 3 months had the intra-articular cavity of his left knee injected with a 60 mg dose of Depo-Medrol (methylprednisolone acetate). On the following day, 8 ml of a solution containing 1% Lidocaine was injected into the intra-articular cavity of this same knee and distributed throughout the joint cavity by bending and straightening of the knee for about 3 minutes prior to intra-articular injection of a 5 ml solution containing 0.2 mg capsaicin. The left knee was vigorously bent for ∼ 30 seconds to distribute the capsaicin fluid within the joint cavity. After 3 minutes from capsaicin injection, the pain level quickly rose to an intolerable level but was immediately diminished by the application of ice packs. Five minutes after the capsaicin injection, the pain level was reduced to a tolerable level. The procedure was concluded in about 23 minutes and the subject expressed complete relief for his painful arthritic symptoms. TABLE XI and Figure 3 include summaries of procedural pain levels as a function of elapsed time.

**TABLE XI**

| **INJECTED KNEE # 3 (Patient #3, left knee treated)** | | |
|---|---|---|
| **ELAPSED TIME (Minutes)** | **PROCEDURE** | **PAIN SCORE (0 -10)** |
| **0** | **Inject 8 cc 1% Lidocaine** | **0** |
| **1** | **Walk, bend Knee, distribute Lidocaine** | **0** |
| **4** | **Inject 0.2 mg Capsaicin** | **0** |
| **5** | **Bend knee vigorously** | **0** |
| **5,5** | **Bend knee vigorously** | **5** |
| **6** | **Bend knee v vigorously** | **6** |
| **6.5** | **Bend knee vigorously** | **8** |
| **7** | **Lying still** | **10** |
| **8** | **Apply ice packs** | **6.5** |
| **9** | **Pain on top of knee** | **5** |
| **10** | **Apply ice packs** | **3** |
| **12** | **Apply ice packs** | **2** |
| **13** | **Apply ice packs** | **1** |
| **13.6** | **Bend knee** | **0.5** |
| **14** | **Ice off** | **0** |
| **14** | **Walk** | **0** |
| **17** | **Pain to ankle** | **2** |
| **18** | **Ice pack applied** | **2** |
| **21** | **Ice pack applied** | **0** |
| **22** | **Bend knee** | **5** |
| **23** | **Ice pack applied** | **0** |
| **25** | | **0** |

### INJECTED KNEE No. 4, PATIENT No. 4

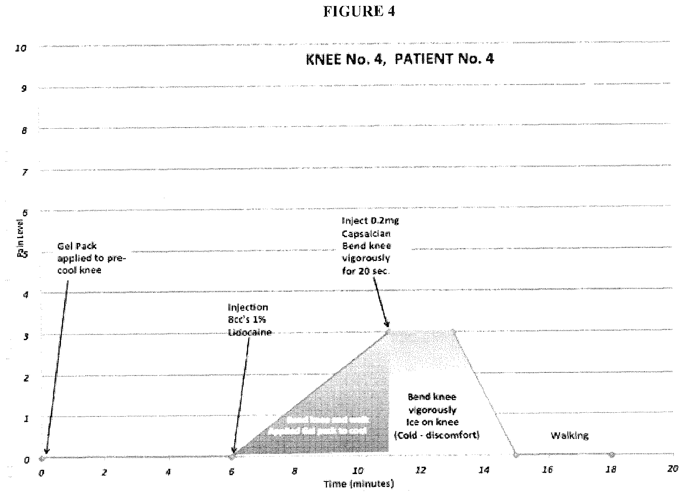

A 66 year old female with severe arthritis in both knees received a 60 mg dose of Depo-Medrol (methylprednisolone acetate) injected into the intra-articular cavity of her left knee. On the following day a gel pack was applied to the knee then 8 ml of a solution containing 1% Lidocaine was injected into the intra-articular cavity of the left knee and distributed throughout the joint cavity by walking and bending of the knee for about 6 minutes prior to intra-articular injection of a 5 ml solution containing 0.2 mg capsaicin. The knee was precooled with a gel pack just prior to the capsaicin injection. The subject noted a mild pain level pain due to the cooling from the gel pack. Immediately after the capsaicin injection the knee was bent vigorously for ∼ 20 seconds to distribute the capsaicin fluid throughout the joint cavity. The pain level remained at a mild level for about 13 minutes and after which the patient experienced complete relief of her painful severe osteoarthritis pain. This patient then requested to have her other knee treated in the same fashion. TABLE XII and Figure 4 include summaries of procedural pain levels as a function of elapsed time.

**TABLE XII**

| **INJECTED KNEE # 4 (Patient # 4, left knee treated with Corticosteroid)** | | |
|---|---|---|
| **ELAPSED TIME (Minutes)** | **PROCEDURE** | **PAIN SCORE (0 - 10)** |
| **0** | **Gel Pack applied to cool knee** | **0** |
| **6** | **Inject 8 cc 1 % Lidocaine, Bend knee & walk, Apply Gel Pack to cool** | **0** |
| **11** | **Inject 0.2 mg Capsaicin, Bend knee vigorously for 20 see ice on knee, cold uncomfortable** | **3** |
| **13** | **Cold uncomfortable, Bend & straighten knee vigorously** | **3** |
| **15** | **-** | **0** |
| **18** | **Walking no pain** | **0** |

### INJECTED KNEE No. 5, PATIENT No. 4

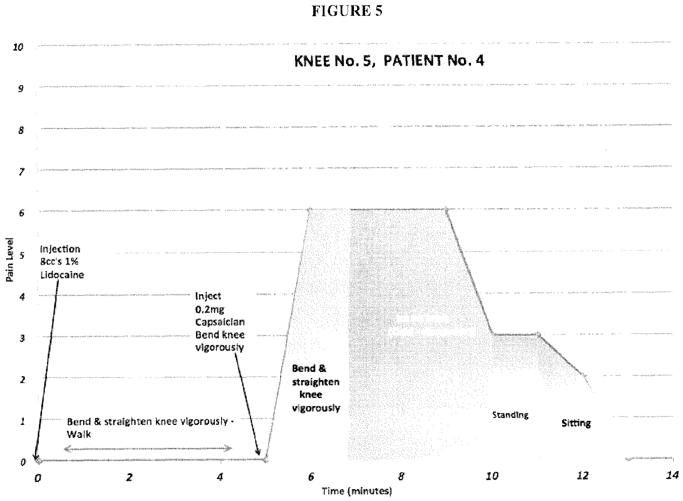

The 66 year old female above requested to have her right knee with severe osteoarthritis treated immediately after her left knee had experienced significant pain relief as a resulted of the capsaicin administration. Noteworthy, this right knee had not previously been treated with corticosteroid (methylprednisolone acetate) injection the previous day. Eight (8) ml of a solution containing 1% Lidocaine was injected into the intra-articular cavity of the right knee and distributed throughout the joint cavity by walking and bending of the knee for about 5 minutes prior to intra-articular injection of a 5 ml solution containing 0.2 mg capsaicin. A moderate level of pain was experienced and an ice pack was applied. The level of pain in her right knee diminished to a mild level and after ∼25 minutes the patient noted that the capsaicin treatment to both knees had alleviated her painful arthritic symptoms and was walking with no further pain. TABLE XIII and Figure 5 includes summaries of procedural pain levels as a function of elapsed time.

**TABLE XIII**

| **INJECTED KNEE # 5 Patient # 4, right knee *not* treated with Corticosteroid)** | | |
|---|---|---|
| **ELAPSED TIME (Minutes)** | **PROCEDURE** | **PAIN SCORE (0 - 10)** |
| **0** | **Inject 8 cc 1% Lidocaine, Bend knee & straighten knee vigorously - walk** | **0** |
| **5** | **Inject 0.2 mg Capsaicin, Bend knee & straighten vigorously** | **0** |
| **6** | **"Feels warm", feels hot spot & ice pack applied** | **6** |
| **9** | **Ice pain** | **6** |
| **10** | **Standing pain** | **3** |
| **11** | **Sitting pain** | **3** |
| **12** | **Standing pain, "Feels warm"** | **2** |
| **13** | **Walking, no further pain** | **0** |

### INJECTED KNEE No. 6, PATIENT No. 1

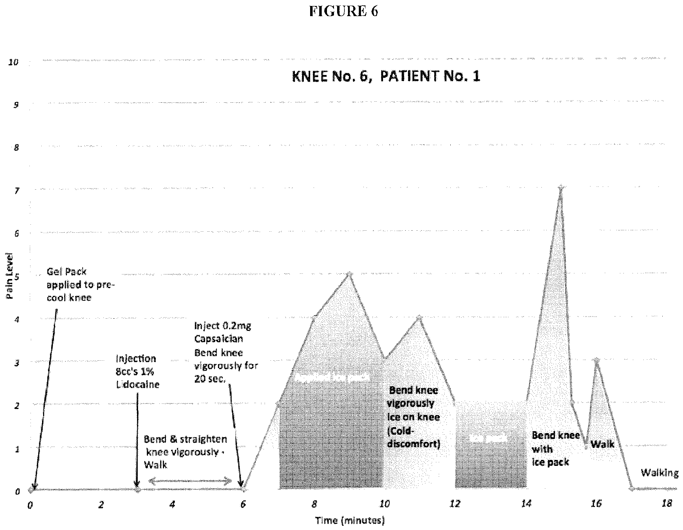

The 76 year old male with painful osteoarthritis of both knees experienced successful capsaicin treatment of the left knee. Three weeks later this patent requested treatment of the right knee The right knee received a 60 mg dose of Depo-Medrol (methylprednisolone acetate) injected into the intra-articular cavity of his right knee. On the following day, a cold pack was applied to this knee and 8 ml of a solution containing 1% Lidocaine was injected and distributed throughout the joint space by bending and straightening the knee and walking for about 3 minutes prior to intra-articular cavity injection of a 5 ml solution containing 0.2 mg capsaicin. The right knee was vigorously bent for ∼ 20 seconds to distribute the capsaicin solution within the joint cavity. Pain levels were held to tolerable levels by the application of ice packs. The procedure was concluded in about 17 minutes and the subject expressed complete relief of his painful arthritic symptoms. The subject expressed satisfaction with procedural differences which resulted in a rapid and extremely tolerable capsaicin administration compared to his previous experience with capsaicin treatment of his left knee. TABLE XIV and Figure 6 include a summary of pain levels as a function of elapsed time.

**TABLE XIV**

| **INJECTED KNEE #6 (Patient # 1, right knee treated)** | | |
|---|---|---|
| **ELAPSED TIME (Minutes)** | **PROCEDURE** | **PAIN SCORE (0-10)** |
| **0** | **Cold pack applied to knee** | **0** |
| **3** | **Inject 8 cc 1% Lidocaine, Bend knee & straighten knee vigorously - walk** | **0** |
| **6** | **Inject 0.2 mg Capsaicin, Bend knee & straighten vigorously for 20 sec.** | **0** |
| **7** | **Gel pack applied to knee** | **2** |
| **8** | **Pain level increased Hard ice applied - hard ice pack more uncomfortable** | **4** |
| **9** | **--** | **5** |
| **10** | **--** | **3** |
| **11** | **Knee bent vigorously, complaint of pain from the cold pack** | **4** |
| **12** | **-** | **2** |
| **14** | **Continuing with ice pack** | **2** |
| **15** | **Bending pain** | **7** |
| **15.3** | **Take off ice pack** | **2** |
| **15.7** | **Reapply ice** | **1** |
| **16** | **Walk** | **3** |
| **17** | **Walking, Completed with no more pain** | **0** |

All patients remain pain free in their treated knees (in one case for over 3 weeks) as of the date of this filing.

## Claims

1. An aqueous pharmaceutical composition comprising:
i) 0.0002% - 0.1 % by weight of a capsaicinoid,
ii) 0.01 % - 1% by weight of menthol and/or eugenol, and
iii) an aqueous vehicle comprising an amount of polyethylene glycol and/or ethylalcohol to solubilize the capsaicinoid and analgesic agent in water, said composition further comprising 0.1%-1.5% by weight of hyaluronic acid.

2. An aqueous pharmaceutical composition as in claim 1, wherein said capsaicinoid is capsaicin.

3. An aqueous pharmaceutical composition as in claim 1, wherein said capsaicinoid is trans- capsaicin.

4. An aqueous pharmaceutical composition as in claim 1, wherein the analgesic agent is menthol.

5. An aqueous pharmaceutical composition as in claim 1, wherein the aqueous vehicle comprises:
i) 15 - 50 % by weight polyethylene glycol, and/or
ii) 0.5 - 40% by weight ethyl alcohol.

6. An aqueous pharmaceutical composition as in claim 1, further comprising 0.01 - 0.1 % by weight phenol.

7. An aqueous pharmaceutical composition as in claim 1 comprising:
0.005 - 0.03 % by weight capsaicin,
0.01 - 0.1 % by weight phenol,
0.01 - 0.1%o by weight menthol,
20 - 35% by weight polyethylene glycol,
5 - 15% by weight ethyl alcohol, and
0.2 - 1 % by weight hyaluronic acid.

8. An aqueous pharmaceutical composition as in claim 1 further comprising an anesthetic agent.

9. A aqueous pharmaceutical composition as in claim 8 wherein said agent is selected from the group consisting of lidocaine, bupivacaine, ropivacaine, dibucaine, procaine, chloroprocaine, prilocaine, mepivacaine, etidocaine, tetracaine, and xylocaine, and mixtures thereof.

10. A composition as claimed in claim 1 for use in treating pain at a site in a mammal by:
administering to said site in said mammal a therapeutically effective amount of the aqueous capsaicinoid composition.

## Patentansprüche

1. Wässrige pharmazeutische Zusammensetzung, umfassend:
i) 0,0002 - 0,1 Gew.-% eines Capsaicinoids,
ii) 0,01 - 1 Gew.-% Menthol und/oder Eugenol, und
iii) ein wässriges Vehikel, das eine Polyethylenglykol- und/oder Ethylalkoholmenge umfasst, um das Capsaicinoid und das analgetische Mittel in Wasser zu solubilisieren,
wobei die Zusammensetzung weiterhin 0,1 - 1,5 Gew.-% Hyaluronsäure umfasst.

2. Wässrige pharmazeutische Zusammensetzung nach Anspruch 1, wobei das Capsaicinoid Capsaicin ist.

3. Wässrige pharmazeutische Zusammensetzung nach Anspruch 1, wobei das Capsaicinoid trans-Capsaicin ist.

4. Wässrige pharmazeutische Zusammensetzung nach Anspruch 1, wobei das analgetische Mittel Menthol ist.

5. Wässrige pharmazeutische Zusammensetzung nach Anspruch 1, wobei das wässrige Vehikel umfasst:
i) 15 - 50 Gew.-% Polyethylenglykol, und/oder
ii) 0,5 - 40 Gew.-% Ethylalkohol.

6. Wässrige pharmazeutische Zusammensetzung nach Anspruch 1, die weiterhin 0,01 - 0,1 Gew.-% Phenol umfasst.

7. Wässrige pharmazeutische Zusammensetzung nach Anspruch 1, umfassend:
0,005 - 0,03 Gew.-% Capsaicin,
0,01 - 0,1 Gew.-% Phenol,
0,01 - 0,1 Gew.-% Menthol,
20 - 35 Gew.-% Polyethylenglykol,
5 - 15 Gew.-% Ethylalkohol, und
0,2 - 1 Gew.-% Hyaluronsäure.

8. Wässrige pharmazeutische Zusammensetzung nach Anspruch 1, die weiterhin ein anästhetisches Mittel umfasst.

9. Wässrige pharmazeutische Zusammensetzung nach Anspruch 8, wobei das Mittel aus der Gruppe ausgewählt ist, die aus Lidocain, Bupivacain, Ropivacain, Dibucain, Procain, Chloroprocain, Prilocain, Mepivacain, Etidocain, Tetracain und Xylocain sowie Gemischen davon besteht.

10. Zusammensetzung nach Anspruch 1, zur Verwendung bei Behandlung von Schmerz an einer Stelle in einem Säuger durch:
Verabreichen, an die Stelle in dem Säuger, einer therapeutisch wirksamen Menge der wässrigen Capsaicinoid-Zusammensetzung.

## Revendications

1. Composition pharmaceutique aqueuse comprenant:
i) 0,0002% - 0,1% en poids d'un capsaïcinoïde,
ii) 0,01% - 1% en poids de menthol et/ou d'eugénol, et
iii) un véhicule aqueux comprenant une quantité de polyéthylèneglycol et/ou d'alcool éthylique pour solubiliser le capsaïcinoïde et un agent analgésique dans de l'eau, ladite composition comprenant en outre 0,1%-1,5% en poids d'acide hyaluronique.

2. Composition pharmaceutique aqueuse selon la revendication 1, dans laquelle ledit capsaïcinoïde est la capsaïcine.

3. Composition pharmaceutique aqueuse selon la revendication 1, dans laquelle ledit capsaïcinoïde est la trans-capsaïcine.

4. Composition pharmaceutique aqueuse selon la revendication 1, dans laquelle l'agent analgésique est le menthol.

5. Composition pharmaceutique aqueuse selon la revendication 1, dans laquelle le véhicule aqueux comprend:
i) 15 - 50% en poids de polyéthylèneglycol, et/ou
ii) 0,5 - 40% en poids d'alcool éthylique.

6. Composition pharmaceutique aqueuse selon la revendication 1, comprenant en outre 0,01 - 0,1% en poids de phénol.

7. Composition pharmaceutique aqueuse selon la revendication 1 comprenant:
0,005 - 0,03% en poids de capsaïcine,
0,01 - 0,1% en poids de phénol,
0,01 - 0,1% en poids de menthol,
20 - 35% en poids de polyéthylèneglycol,
5 - 15% en poids d'alcool éthylique, et
0,2 - 1% en poids d'acide hyaluronique.

8. Composition pharmaceutique aqueuse selon la revendication 1 comprenant en outre un agent anesthésique.

9. Composition pharmaceutique aqueuse selon la revendication 8 dans laquelle ledit agent est sélectionné dans le groupe constitué par la lidocaïne, la bupivacaïne, la ropivacaïne, la dibucaïne, la procaïne, la chloroprocaïne, la prilocaïne, la mépivacaïne, l'étidocaïne, la tétracaïne, et la xylocaïne, et leurs mélanges.

10. Composition selon la revendication 1 destinée à être utilisée dans le traitement de la douleur au niveau d'un site chez un mammifère par:
administration audit site chez ledit mammifère d'une quantité thérapeutiquement efficace de la composition aqueuse de capsaïcinoïde.
